# EUROPEAN PATENT APPLICATION

(11) **EP 4 516 739 A1**
(43) Date of publication of application: **05.03.2025**
(21) Application number: 23194320.0
(22) Date of filing: 30.08.2023
(51) Int. Cl.: C01C 1/04, C07C 29/152

(54) **CONTROL OF AMMONIA OR METHANOL SYNTHESIS LOOP AT PARTIAL LOAD**

(71) Applicant: CASALE SA, 6900 Lugano (CH)
(72) Inventor: PANZA, Sergio, 22100 Como (IT); PIN, Mattia, 22100 Como (IT); CARRARA, Davide, 22073 Fino Mornasco (CO) (IT); GENOVA, Giovanni, 22100 Como (IT)
(74) Representative: M. Zardi & Co S.A.

(57) **Abstract**

A method for controlling an ammonia synthesis loop (101) or a methanol synthesis loop at partial load, wherein the loop includes a circulator (4), a first differential pressure controller (DPC1) responsive to a differential pressure between the suction side and the delivery side of said circulator (4); a loop control valve (5, 6) arranged to control the pressure of the loop and/or the flow rate which circulates in the loop, wherein the opening of said first loop control valve is governed by said differential pressure controller to maintain a target pressure of the loop even at partial load.

## Description

### Field of application

The invention relates to the field of industrial synthesis of ammonia and industrial synthesis of methanol. The invention pertains to a method for controlling a synthesis loop at partial load.

### Prior art

The industrial production of ammonia includes the generation of a make-up gas (MUG) in the front-end and the conversion of said makeup gas in a so-called ammonia synthesis loop. Similarly, the production of methanol includes the generation of a suitable make-up gas and the conversion in a synthesis loop.

The synthesis loop includes a catalytic converter wherein the makeup gas is reacted under appropriate temperature and pressure. The effluent of the converter typically contains unconverted reagents which are separated and recycled to the converter thus forming the synthesis loop.

The synthesis loop operates at a pressure which is typically much higher than the pressure of the makeup gas generated in the front-end. A main compressor elevates the pressure of the make-up gas to feed the loop. A circulator stage compressor maintains circulation in the loop and compensates for the pressure losses in the piping of the loop. The circulator can be a stand-alone machine or in some cases the circulator and the main compressor are mounted on the same shaft.

Both the synthesis of ammonia and synthesis of methanol rely basically on the production of hydrogen. The ammonia make-up gas is essentially a mixture of hydrogen and nitrogen in a suitable proportion, wherein nitrogen may be added with combustion air in a secondary reformer or separately when available, e.g. from an air separation unit. In methanol synthesis, the make-up gas is essentially a mixture of hydrogen and carbon monoxide.

When the hydrogen for the make-up gas comes from a fossil source (reforming of hydrocarbons, such as natural gas, or gasification of coal), the production of hydrogen is nearly constant and the synthesis loop can generally be operated steadily at or near 100% of the nominal capacity (also termed plant capacity). Therefore, in conventional fossil fuel-based facility the flexibility of the loop, that is the ability to operate at partial load, is generally not an issue.

Recently, a great interest emerged about applications wherein at least part of the hydrogen is sourced from renewable energy such as solar or wind. A particularly interesting application is the production of hydrogen via water electrolysis powered by wind turbines or solar cells. Accordingly, the feed of the hydrogencontaining make up gas fluctuates following the availability of the renewable energy (sunlight and/or wind).

A hydrogen storage may compensate, at least temporarily, for the lack of renewable energy, but storage is expensive. There is therefore the need to develop new methods to control the synthesis loop, in order to cope with the fluctuations of the hydrogen feed. In practice, this requires the ability to operate the ammonia plant or the methanol plant at a partial load significantly less than 100%, such as less than 50% or even less than 20%, for example at 10% of the nominal load.

A problem encountered when trying to operate the synthesis loop at a low load is that the operation of the circulation compressor may become unstable. Additionally, the pressure in the converter should be kept sufficiently high to maintain the conversion reaction and allow the converter to rapidly return at full load when the hydrogen feed is restored. A further problem connected with operation at variable load is that cyclic variation of the operating parameters of the synthesis loop such as the loop operating pressure or temperature may induce fatigue stress of the equipment.

A shut-down of the converter is not desired because it requires time to restart and may cause fatigue stress. At a low load, the reaction may not produce a sufficient heat to maintain operation of the converter. The converter may be equipped with a start-up heater, as it is typically the case of ammonia converters. A start-up heater, at least theoretically, might be used at low load to provide additional heat for operation; this solution however requires additional energy which may not be available or may be expensive, and for this reason is not attractive.

The circulator stage compressor is normally equipped with internal controls adapted to avoid mechanical damage possibly caused by operation at a low or very low load. For example, a centrifugal compressor is normally equipped with an anti-surge valve. Said valve however is designed to intervene in limited cases to protect the machine and there is the need of a more accurate control which takes into account not only the operation of the compressor as such but also the operation of the entire loop.

In summary, operating an ammonia synthesis loop or a methanol synthesis loop at partial load is challenging. Solutions for controlling the loop at partial load have been proposed in EP 3 819 261 and WO 2021 233 780 but there is still the need to improve the state of the art.

### Summary of the invention

The invention aims to provide a method of controlling an ammonia synthesis loop or a methanol synthesis loop in a broad range of operating loads. The invention addresses the problem of how to control the synthesis loop in order to maintain stable operation of the circulation compressor and sufficient pressure of the ammonia or methanol converter, when the hydrogen feed is significantly less than the nominal value.

The problem is solved with a method according to the claims.

The invention uses at least a first differential pressure controller responsive to a differential pressure between a first point of measure located at the suction side of the loop circulator and a second point of measure located at the delivery side of said circulator.

Said differential pressure controller governs a loop control valve arranged to control the pressure of the loop and/or the flow rate in the loop. A setpoint is provided to said differential pressure controller so that the opening of said loop control valve is controlled according to said setpoint.

The location of said loop control valve may differ according to various embodiments of the invention. In preferred embodiments said loop control valve is an admission valve of the circulator or is a main feed valve on a main feed line directed to the converter. The loop may be additionally controlled by one or more other valve(s) according to embodiments of the invention.

The method of the invention delivers a stable operation of the loop even at low loads, allowing the loop to reach a low percentage of the nominal load, such as 20% or 10% or 5% of the nominal load, without instability of the compressor and maintaining an acceptable pressure of the loop.

### Description of the invention

The invention is directed to controlling an ammonia synthesis loop or a methanol synthesis loop running at partial load. The loop receives a fresh make-up gas produced in a suitable front-end. The loop has a full load condition corresponding to a nominal input of fresh make-up gas, and a partial load is a condition wherein the loop operates with an input of fresh makeup gas less than said nominal input.

The load of the synthesis loop may be denoted by the amount of make-up gas converted to ammonia or methanol.

In a preferred embodiment, at least part of the energy required for the production of the make-up gas comes from a renewable source, to reduce the carbon footprint of the process. The term renewable source, according to the common definition, denotes any source which is naturally replenished, including biomass. In a highly preferred embodiment, some or all of the hydrogen in the make-up gas is produced by solar-powered or wind-powered water electrolysis or by a blend of the previous.

The synthesis loop includes typically a converter wherein a feed gas is reacted; a circulation compressor configured to maintain circulation in the synthesis loop; a line arranged to feed said converter and including a main feed valve. The circulator compressor can be of any type, such as reciprocating or centrifugal.

In addition, the loop normally comprises a cooling and separation stage wherein a liquid product (ammonia-containing or methanol-containing liquid product) is obtained from cooling and condensation of the hot converter effluent. The separation process produces a stream of unreacted gas which is returned to the suction side of the circulator and mixed with the fresh gas from the front-end, to form the feed of the converter. Accordingly, the feed gas reacted in the converter includes the fresh make-up gas and a recirculated portion of unreacted gas.

In a broad aspect of the invention, at least a first differential pressure controller is provided, which is responsive to a differential pressure between a first point of measure located at the suction side of said circulator and a second point of measure located at the delivery side of said circulator; at least a first loop control valve is arranged to control the pressure of the loop and/or the flow rate which circulates in the loop; said first loop control valve is controlled by said first differential pressure controller; the method of controlling the loop at partial load includes providing a setpoint to said differential pressure controller so that the opening of said first loop control valve is controlled according to said setpoint.

The setpoint to said first differential pressure controller may be a static setpoint or a dynamic setpoint in relation to the load of the synthesis loop. A static setpoint does not depend on the load whereas a dynamic setpoint is continuously adjusted according to the instant load of the loop. The load of the loop may be represented by the fresh make up gas sent to the loop, or by the plant production.

The set point to said first differential pressure controller may be inserted manually or provided by another controller arranged in cascade. Said another controller is preferably a pressure controller at a selected location to detect the pressure in the loop. In addition to providing the setpoint to the differential pressure controller, said pressure controller may control one or more other valves of the loop. Said pressure controller is preferably arranged to detect the pressure in a feed line of the converter.

In an embodiment, the method includes the step of adjusting the setpoint to said first differential pressure controller in response to a variation of the pressure of the loop, detected by said pressure controller, which is greater than a selected threshold. Said threshold may be a variation of 5%, 10%, 15% according to certain embodiments.

Said first loop control valve can be governed to maintain a target loop pressure or to avoid that loop pressure falls below a predetermined value. Said target difference of pressure, in some embodiments, is a constant difference of pressure in the range of 1.0 to 10.0 bar. In certain embodiments said difference may be in a narrower range such as 1.0 to 8.0 bar or 1.0 to 5.0 bar or 1.0 to 2.0 bar or 1.4 to 1.6 bar. A relatively broad range having an upper limit such as 8 bar or 10 bar may be preferred to control the loop across a wide variation of load, such as 10% to 110%, with a single control valve. A control with a single valve is preferably carried out using the converter main feed valve or the compression suction valve.

In some embodiments, the loop pressure is controlled to keep the operating pressure of the loop within a range, said range being preferably determined as a percentage of the design pressure. The design pressure denotes the maximum pressure for the equipment.

In preferred embodiments, the synthesis loop is additionally controlled by varying the opening of one or more additional valves of the loop. Preferably said one or more valves are governed by one or more pressure controllers. The pressure controller or each pressure controller may have a setpoint which correspond to a target pressure to be maintained in the loop at partial load, or at least the operating pressure is maintained within a specific range. Preferably said range is maximum 15% of design pressure, for example operating pressure range +/-7.5% of the design pressure. As an example, design pressure is 175 bar gauge; operating pressure is 145 bar gauge; said range is +/- 13 bar. Said setpoint may be fixed or variable.

In some embodiments, the invention makes use of a split-range technique. Accordingly, a signal provided by a controller can be split to control two or more valves of the loop. A split-range control may be applied to a pressure controller or to the differential pressure controller of the main feed valve.

The above-mentioned loop control valve, governed by said first differential pressure controller, may be provided at various locations of the loop. In a preferred embodiment, said valve is an admission valve located at the suction side of the circulator. In another preferred embodiment, said loop control valve is a feed valve of the converter.

A circulator admission valve is a valve located at the suction side of said compressor. By varying the opening of said valve, the circulator inlet pressure varies. As a consequence, the circulator operates accordingly to its type (centrifugal or reciprocating) and characteristic curves.

Said first differential pressure controller is preferably arranged to detect the pressure in the proximity of the delivery side and in the proximity of the suction side of the circulator. Said first point of measure and second point of measure are preferably close to the delivery side and suction side of the circulator, so that the differential pressure controller responds to the difference of pressure (delta-P) across the circulator.

Preferably said first point of measure is located between the cooling-separation stage and the circulator, and said second point of measure is located between the circulator and a feed gas pre-heating stage. Accordingly, there is no substantial equipment between the circulator and the points of measure, and the difference sensed by the controller corresponds to the difference of pressure between delivery and suction of the circulator.

In some embodiments, the loop is controlled by the combined action of said loop control valve and at least another valve.

In addition to said first loop control valve, the loop may include a second loop control valve governed by a second differential pressure controller. In such a case, the method includes providing a setpoint to said second differential pressure controller.

In a preferred embodiment, the first loop control valve is an admission valve of the circulator and the second loop control valve is located on a main feed line directed to the converter. More preferably, said second differential pressure controller is responsive to difference of pressure across said second loop control valve, namely to a difference of pressure between a point of said main feed line upstream the second loop control valve and a point of said main feed line downstream said second loop control valve.

In a further preferred embodiment, the opening of said second loop control valve is controlled to maintain a target difference of pressure across the same valve.

Some embodiments may include also a converter bypass valve. A converter bypass valve is understood as a valve on a converter bypass line connecting a point in the loop downstream of said circulator to a point in the loop upstream of said circulator and arranged so that a portion of the feed gas delivered by the circulator can be sent back to the suction of the circulator, travelling through said converter bypass line and without passing through the converter. In some embodiments said bypass valve is in addition to an anti-surge valve (or kick-back valve) of the circulation compressor. In some embodiments, an anti-surge valve or kick-back valve of the circulation compressor is used as bypass valve.

Some embodiments may include a converter feed secondary valve provided on a secondary feed line in parallel to the main feed line of the converter. The secondary line and the related secondary valve are generally smaller than the main line / main valve. When installed, the secondary feed valve is preferably governed by a differential pressure controller of the main valve.

In certain embodiments of the invention, one or more of said converter bypass valve, compressor admission valve and converter feed secondary valve is governed by a pressure controller responsive to the pressure in the loop. The method of controlling the pressure in the loop at partial load may include the provision of a setpoint to said pressure controller to control the opening of said valve(s).

In the various embodiments of the invention, a pressure controller is preferably located on a line connecting the delivery of the circulation compressor to the inlet of the converter. The same pressure controller or different pressure controllers may be arranged to control the converter by-pass valve and/or the compressor admission valve and/or the converter main feed valve.

In an interesting embodiment, a pressure controller governs the converter bypass valve and provides the setpoint to the differential pressure controller of the converter main feed valve. In a variant of this embodiment, the differential pressure controller controls the main feed valve and secondary feed valve of the converter in a split-range.

In preferred embodiments of the invention, the synthesis loop reaches a partial load of less than 50% of the nominal load, preferably less than 20%, more preferably until around 10% and further more preferably about 5%.

Another aspect of the invention is a synthesis section for producing ammonia or methanol according to the claims, including a synthesis loop and a control system configured to operate with the method of the invention.

The method of the invention enables effective control of the synthesis loop in a broad range of load conditions. The method of the invention keeps the circulation compressor from operating under unstable conditions. The invention avoids the shutdown of the converter at reduced loads and avoids the need for using the start-up heater of the converter at low capacity. Also, the invention provides a flexible control which is adapted to drive the loop from a very low load, such as 10% or even less, to a full load of 100% or, when possible, exceeding nominal capacity, such as 110%.

### Description of the figures

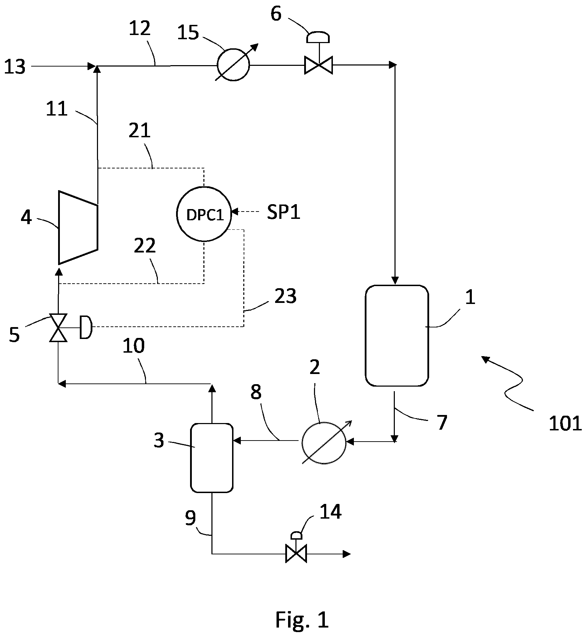

Figs. 1 to 3 are schematic diagrams of a synthesis loop for the preparation of ammonia or methanol and will be used to illustrate some embodiments of the method of the present invention.

Fig. 1 illustrates a synthesis loop 101 with the following components and features:
1 Converter
2 Condensing system of the converter effluent
3 Separator
4 Circulation compressor ("circulator")
5 Admission valve o of the circulator 4
6 Feed valve of the converter 1 ("main valve")
7 Hot gas effluent from the converter 1
8 Cooled effluent
9 Liquid product separated in the separator 3 and containing ammonia or methanol
10 Unreacted gas withdrawn from the separator 3 and recycled to the converter 1
11 Syngas stream delivered by the circulator 4
12 Feed line directed to the converter 1
13 Fresh gas input
14 Liquid output valve
15 Pre-heating stage of the feed gas directed to the converter 1

DPC1 differential pressure controller
SP1 set point of the controller DPC1
21 input signal of the pressure in the line 11 (delivery side of circulator 4) to the controller DPC1
22 input signal of the pressure in the line 10 (suction side of the circulator 4) to the controller DPC1
23 output signal sent from the controller DPC1 to the admission valve 5, controlling the opening of the valve 5.

The liquid product which contains ammonia or methanol is withdrawn at line 9. The liquid level in the separator 3 is controlled by a suitable level controller, governing the valve 14.

The unreacted gas in line 10 is sent to the converter 1 via the circulator 4, together with the fresh gas 13. A gas-gas heat exchanger (not shown) is optionally provided on the line 10 to heat the unreacted gas above the dew point.

The fresh make-up gas entering at line 13 is provided by a front-end and is elevated to synthesis pressure by a suitable make-up gas compressor (not shown).

The fresh make-up gas 13 may be introduced into the feed line 12 at the discharge side of the circulator 4, as shown, or at a different location, for example at the suction side of the circulator 4.

Fig 1 illustrates a first embodiment of the invention the differential pressure controller DPC1 governs the admission valve 5 of the circulator 4. The degree of opening of said valve 5 is controlled on the basis of the difference of pressure across the circulator 4 and on the basis of the setpoint SP1.

The differential pressure controller DPC1 is responsive to a differential pressure between the suction side of said circulator and the delivery side of said circulator. The pressure detected at the delivery side of the circulator is transmitted to the controller via the line 21 and the pressure detected at the suction side is transmitted via line 22.

In Fig. 1, the admission valve 5 is operated as a loop control valve to control the pressure of the loop 101. In this embodiment, the setpoint SP1 is provided to said differential pressure controller DPC1 so that the opening of said admission valve 5 is controlled and, consequently, the pressure in the loop 101 is controlled when the loop 101 is running at a partial load, for example when the input 13 is significantly smaller compared to nominal condition.

Preferably the points of connection of the differential pressure controller DPC1 are next to the circulator 4 without other items in between. Accordingly, the difference of pressure detected by said controller DPC1 is substantially the pressure drop across the circulator 4.

The output signal of said controller DPC1 is transmitted to the admission valve 5 via line 23 and governs the opening of said valve 5. The set point SP1 can be constant or dependent on the current load of the loop according to different embodiments. In some embodiments, the set point SP1 depends on one or more of the following: the amount of the fresh make up gas to the loop; the plant production; a variation of the loop operating pressure. In the latter case, the system is preferably configured to avoid a significant change of the loop operating pressure.

In a preferred embodiment, the set point SP1 to the controller DPC1 is provided by a pressure controller of the feed line 12, located upstream or downstream the pre-heating stage 15.

In a preferred embodiment, the pressure in the loop is controlled solely by a valve, which is preferably the main valve 6 or the compressor suction valve 5. In such a case the target difference of pressure of the controller DPC1 is preferably set to a value in the range 1 to 10 bar.

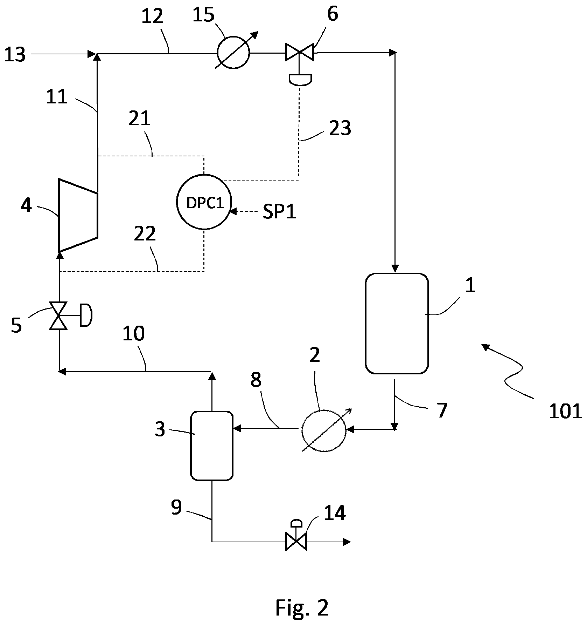

Fig. 2 illustrates an embodiment similar to Fig. 1 wherein the output signal 23 of the controller DPC1 governs the opening of said main feed valve 6. Accordingly, the loop pressure is controlled by means of said feed valve 6 instead of the admission valve 5.

In summary, Fig. 1 and Fig. 2 show embodiments wherein the loop pressure at a partial load is controlled by a loop control valve, which is either the admission valve 5 or the main feed valve 6, and said loop control valve is governed by the differential pressure controller DPC1.

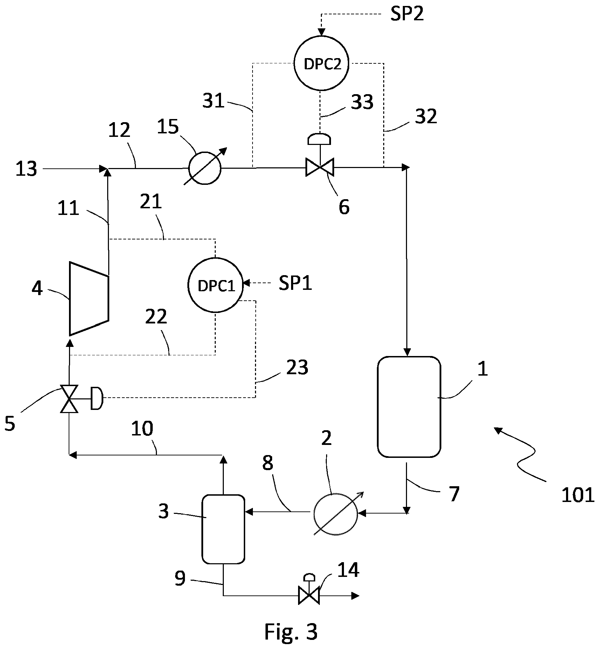

Fig. 3 illustrates an embodiment wherein the admission valve 5 is governed by the differential pressure controller DPC1, similar to Fig. 1 whereas the main feed valve 6 is governed by a second differential pressure controller DPC2 responsive to the difference of pressure across the same valve 6. Said second controller DPC2 has a setpoint SP2 which may be fixed or variable, according to the load of the loop, similarly to the above-described setpoint SP1.

In a preferred embodiment, the second controller DPC2 is configured to maintain a constant difference of pressure across the feed valve 6. The setpoint DPC2 can be constant or variable within a range, said range being preferably not greater than +/- 0.5 bar and more preferably +/- 0.2 bar.

The setpoint SP1 in other embodiments can be determined as a function of the plant load or of the make-up gas flow.

## Claims

1. A method for controlling an ammonia synthesis loop (101) or a methanol synthesis loop running at partial load wherein:
a fresh make-up gas (13) is fed to said synthesis loop;
the synthesis loop has a full load condition corresponding to a nominal input of said fresh make-up gas, and a partial load is a condition wherein the loop operates with an input of fresh makeup gas less than said nominal input;
said synthesis loop includes:
a converter (1) wherein a feed gas is reacted;
a circulator (4) which is a compressor configured to maintain circulation in the synthesis loop;
a first differential pressure controller (DPC1) responsive to a differential pressure between a first point of measure located at the suction side of said circulator (4) and a second point of measure located at the delivery side of said circulator;
at least a first loop control valve (5, 6) arranged to control the pressure of the loop and/or the flow rate which circulates in the loop, wherein said first loop control valve is controlled by said differential pressure controller;
wherein the method of controlling the loop at partial load includes the steps of providing a setpoint (SP1) to said first differential pressure controller (DPC1) so that the opening of said first loop control valve (5, 6) is controlled according to said setpoint.

2. A method according to claim 1 wherein said loop control valve is an admission valve (5) of the circulator (4) located at the suction side of said circulator.

3. A method according to claim 1 wherein said loop control valve is a feed valve (6) of the converter (1).

4. A method according to any of claims 1 to 3 wherein the loop includes:
a heating stage (15) located upstream the converter (1) arranged to preheat a feed gas delivered by the circulator (4) and directed to the converter (1);
a cooling-separation stage (2, 3) downstream the converter (1) arranged to process the effluent of the converter obtaining a liquid product and unreacted gas which is sent to the circulator;
wherein said first point of measure of said first differential pressure controller (DPC1) is located between the cooling-separation stage and the circulator, and said second point of measure is located between the circulator and said heating stage.

5. A method according to any of the previous claims wherein said setpoint (SP1) is a constant value or is variable depending on the load of the loop.

6. A method according to any of the previous claims wherein said setpoint (SP1) of the first differential pressure controller is determined as a function of the plant load or as a function of the make-up gas flow.

7. A method according to any of the previous claims wherein said setpoint (SP1) is provided by another controller arranged in cascade with said differential pressure controller.

8. A method according to claim 7 wherein said another controller is a pressure controller responsive to the pressure in a selected location of the loop, preferably to the pressure in a feed line (12) of the converter.

9. A method according to any of the previous claims wherein the loop, in addition to said first loop control valve, includes a second loop control valve controlled by a second differential pressure controller (DPC2), and the method includes providing a setpoint (SP2) to said second differential pressure controller.

10. A method according to claim 9 wherein said first loop control valve is an admission valve (5) of the circulator (4) and said second loop control valve is a feed valve (6) of the converter (1).

11. A method according to claim 10, wherein said second differential pressure controller (DPC2) is responsive to difference of pressure between a point of a converter feed line located upstream said feed valve (6) and a point of said feed line located downstream said valve.

12. A method according to claim 11 wherein the opening of said feed valve (6) is controlled to maintain a target difference of pressure across the same valve.

13. A method according to claim 12, wherein said target difference of pressure is a constant difference of pressure in the range of 1.0 to 10.0 bar, preferably 1.0 to 5.0 bar or 1.0 to 2.0 bar and more preferably 1.4 to 1.6 bar.

14. A method according to any of the previous claims including the step of adjusting the setpoint to said first differential pressure controller (DPC1) in response to a variation of the pressure of the loop which is greater than an adjustment threshold, said adjustment threshold being preferably a variation of 5% or 10% or 15%.

15. A method according to any of the previous claims wherein the loop pressure is controlled to keep the operating pressure of the loop within a range, said range being preferably 15% of the design pressure.

16. A synthesis section for the synthesis of ammonia or methanol, including a fresh make-up gas input, a converter, a separator where the effluent of the converter is separated into a liquid product and unreacted gas, a line arranged to reintroduce the unreacted gas into the converter, together with the fresh make-up gas, to form a synthesis loop, a circulation compressor adapted to maintain circulation within the loop and to feed the converter, said synthesis section further including a control system configured to control the loop in accordance with the method of any of claims 1 to 15.
